# FASCICULE DE BREVET EUROPEEN

(11) **EP 4 386 079 B1**
(45) Date de publication et mention de la délivrance du brevet: **20.11.2024**
(21) Numéro de dépôt: 23209959.8
(22) Date de dépôt: 15.11.2023
(51) Int. Cl.: C12M 1/32, C12M 3/06, C12M 1/12, C12M 1/00, C12M 1/42, C12M 3/00

(54) **DISPOSITIF DE CULTURE CELLULAIRE AVEC FONCTION DE STIMULATION MÉCANIQUE**
ZELLKULTURVORRICHTUNG MIT MECHANISCHER STIMULATIONSFUNKTION
CELL CULTURE DEVICE WITH MECHANICAL STIMULATION FUNCTION

(30) Priorité: 15.12.2022 FR 2213409
(43) Date de publication de la demande: 19.06.2024
(73) Titulaire: Commissariat à l'Energie Atomique et aux Energies Alternatives, 75015 Paris (FR)
(72) Inventeur: LAPORTE, Camille, 38054 Grenoble cedex 09 (FR); REVOL-CAVALIER, Frédéric, 38054 Grenoble cedex 09 (FR); DEN DULK, Remco, 38054 Grenoble cedex 09 (FR); COSNIER, Marie-Line, 38054 Grenoble cedex 09 (FR)
(74) Mandataire: INNOV-GROUP

(56) Documents cités:
- WO-A1-01/53451
- WO-A1-2018/096054
- DE-A1- 102009 057 698

## Description

### Domaine technique de l'invention

La présente invention se rapporte à un dispositif de culture cellulaire avec fonction de stimulation mécanique.

### Etat de la technique

Les organes et tissus d'un être vivant sont soumis à différents stimuli mécaniques tels que des contraintes de cisaillement dans les vaisseaux, de flux interstitiel, de compression ou au contraire d'étirement. La reproduction de ces contraintes biomécaniques dans des microsystèmes, au même titre que l'intégration du microenvironnement des cellules, est cruciale à l'établissement d'un modèle d'organe sur puce physiologique et fonctionnel.

Les organes sur puces ou *"organ-on-chip"* (OOC) permettent de récréer *in vitro,* la complexité de l'organisation tridimensionnelle et des stimuli chimiques et mécaniques propres à un organe spécifique. Un OOC est une plateforme microfluidique destinée à accueillir et maintenir en culture un objet biologique multicellulaire imitant l'architecture 3D physiologique d'un tissu ou d'un organe. Cette plateforme permet de contrôler le microenvironnement de l'objet biologique, notamment en assurant une perfusion de type vasculaire.

La fonction de stimulation mécanique d'un modèle de peau sur puce semble particulièrement pertinente au regard des limites des modèles actuels de peaux reconstruites. Les modèles de peaux actuels échouent à reproduire les paramètres de perméabilité de la peau et donc sa fonctionnalité. Les peaux *in vitro* sont beaucoup plus perméables à différentes molécules, ce qui constitue un frein à leur utilisation pour les tests de pénétration, irritation et sensibilisation des produits médicamenteux et cosmétiques.

La demande de brevet WO2018/096054A1 décrit un système à double membrane élastique permettant de reproduire les mouvements du diaphragme et des cycles d'extension/contraction des poumons. Le système utilise une membrane de culture micro-perforée pour perfuser les tissus stimulés et une membrane d'actionnement qui vient indirectement solliciter la membrane de culture via le milieu de culture en augmentant la pression dans le circuit utilisé pour le milieu de culture.

Cependant, cette solution présente certains inconvénients :
- Le risque de décollement des tissus soumis à la stimulation mécanique ;
- Le risque d'une fuite de fluide (liquide ou gaz) à travers la membrane d'actionnement ;
- Les limitations mécaniques du matériau en PDMS utilisé pour réaliser la membrane de culture ;

Le but de l'invention est de proposer un dispositif de stimulation adapté pour pallier les inconvénients de l'état de la technique.

### Exposé de l'invention

Ce but est atteint par un dispositif de culture cellulaire doté d'une fonction de stimulation mécanique de tissus biologiques, comprenant :
- Un composant fluidique dans lequel est réalisé un puits qui est creusé selon une direction dite principale,
- Une première membrane poreuse déformable élastiquement, dite membrane de culture, intégrée audit composant et s'étendant transversalement à ladite direction principale pour obturer ledit puits, ladite membrane de culture comprenant une face supérieure destinée à recevoir une culture cellulaire et une face inférieure opposée,
- Un premier circuit fluidique intégré audit composant, comprenant une première cavité réalisée dans ledit puits sous ladite membrane de culture et destinée à recevoir un milieu de culture adapté à la perfusion desdits tissus biologiques,
- Une deuxième membrane non poreuse, dite membrane d'actionnement, déformable élastiquement, intégrée audit composant,
- Des moyens d'actionnement de ladite membrane d'actionnement,
- La membrane d'actionnement étant agencée de manière à fermer le fond de ladite première cavité de manière étanche, et configurée pour se déformer sous l'action desdits moyens d'actionnement vers une position dans laquelle elle pousse mécaniquement ladite membrane de culture pour la déformer.

Selon une réalisation avantageuse, la membrane de culture est une mousse tridimensionnelle d'épaisseur constante suivant la direction principale.

Selon une particularité, la mousse tridimensionnelle est structurée sur sa face supérieure de manière à présenter des variations d'épaisseur suivant la direction principale. Selon une autre particularité, la mousse est réalisée dans un matériau choisi parmi le polyéthylène, le polyuréthane, le polychlorure de vinyle, le caoutchouc.

Selon une autre réalisation, la membrane de culture est une bande de matière perforée, agencée transversalement à ladite direction principale. La membrane est alors par exemple réalisée en PDMS.

Selon une particularité, les moyens d'actionnement de la membrane d'actionnement comportent un deuxième circuit fluidique d'actionnement, intégré audit composant et comprenant au moins une deuxième cavité de mise en pression située sous la membrane d'actionnement et destinée à recevoir un fluide.

Selon une autre particularité, le fluide est un liquide ou un gaz.

L'invention concerne également un système de culture cellulaire qui comporte plusieurs dispositifs de culture cellulaire à fonction de stimulation mécanique tels que définis dans ci-dessus, lesdits dispositifs étant juxtaposés au sein d'un même composant fluidique.

### Brève description des figures

D'autres caractéristiques et avantages vont apparaître dans la description détaillée qui suit, en liaison avec les figures ci-dessous :
- Les figures 1A à 1C montrent un premier exemple de réalisation du dispositif de l'invention, respectivement avant stimulation, avec les membranes à l'état repos et à l'état déployé ;
- Les figures 2A à 2C montrent un deuxième exemple de réalisation du dispositif de l'invention, respectivement avant stimulation, avec les membranes à l'état repos et à l'état déployé ;
- Les figures 3A à 3C montrent un troisième exemple de réalisation du dispositif de l'invention, respectivement avant stimulation, avec les membranes à l'état repos et à l'état déployé ;
- La figure 4 montre une solution avantageuse d'un système comportant plusieurs dispositifs juxtaposés au sein d'un même composant ;

### Description détaillée d'au moins un mode de réalisation

Le dispositif de l'invention vise à permettre une stimulation mécanique de tissus mis sous perfusion d'un milieu de culture.

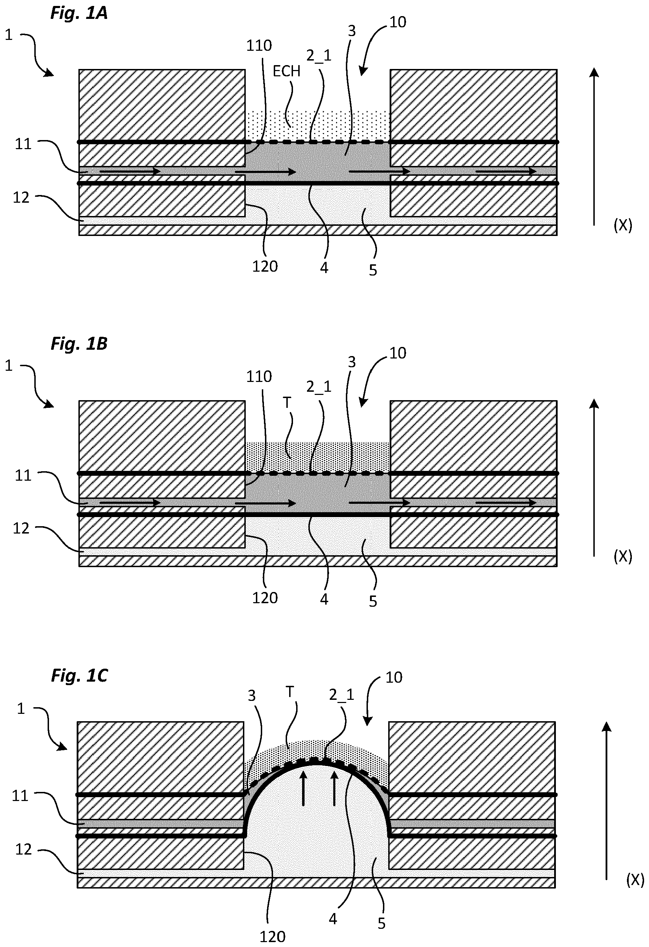

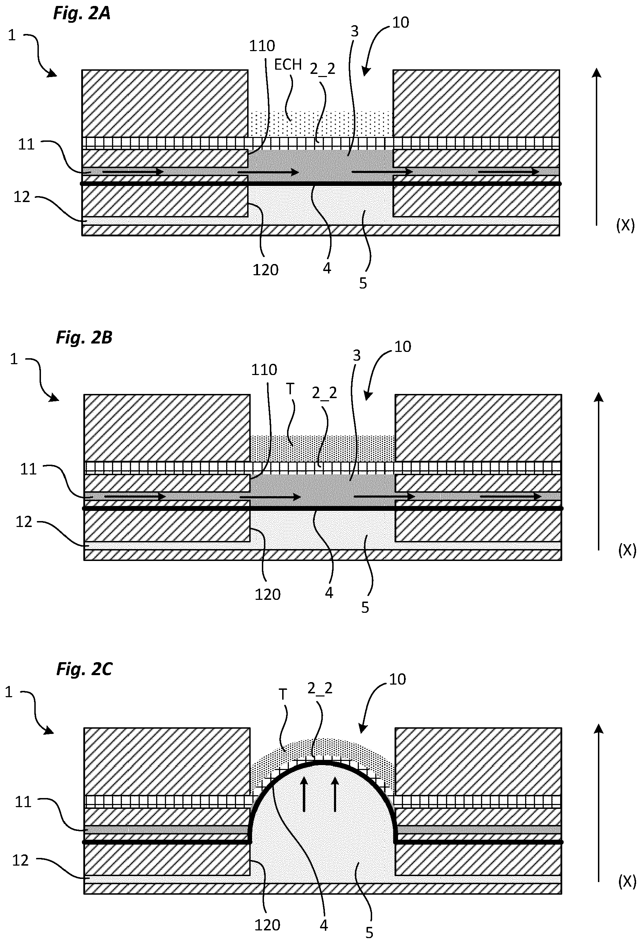

L'invention est d'abord décrite ci-dessous en liaison avec la figure 1A, la figure 1B et la figure 1C, et en liaison avec la figure 2A, la figure 2B et la figure 2C.

Le dispositif comporte un composant 1 fluidique. Ce composant 1 fluidique peut être réalisé par l'assemblage de plusieurs couches. Il peut être réalisé dans un matériau tel que par exemple le COC (Cyclic Olefin Copolymer), le verre ou le PMMA (Polyméthacrylate de Méthyle).

Les différentes couches du composant 1 sont assemblées suivant une direction, appelée ci-après direction principale (X) orientée vers le haut de la feuille. Cette direction principale (X) est verticale lorsque le composant est posé sur un support. Les termes "supérieur", "inférieur", "haut", "bas", "au-dessus" et "au-dessous" sont à comprendre en tenant compte de cette direction principale.

Un puits 10 est creusé dans ledit composant 1, suivant ladite direction principale (X), de manière à traverser plusieurs couches du composant. Le puits 10 est ouvert du côté supérieur du composant 1 et fermé du côté inférieur du composant.

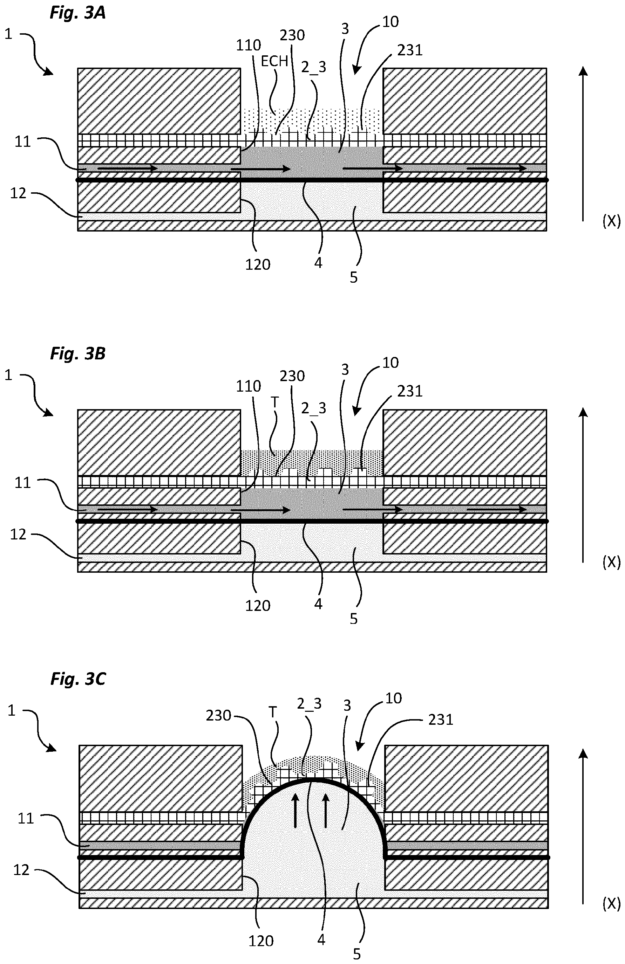

Le dispositif comporte une première membrane 2_1, 2_2 poreuse déformable, intégrée dans le composant 1, venant obturer le puits 10 du côté supérieur, en s'étendant transversalement à la direction principale (X), ceci tout en laissant un espace de dépôt au-dessus d'elle. Cette membrane 2_1, 2_2 est la membrane de culture, c'est-à-dire qu'elle est le siège de la culture cellulaire. Elle comporte ainsi une face supérieure accessible par le haut du puits 10 et une face inférieure, opposée. Sa face supérieure est destinée à recevoir une culture cellulaire (échantillon ECH de culture cellulaire déposé initialement sous forme de liquide ou de gel dans le puits 10, comme représenté sur la figure 1A, la figure 2A et sur la figure 3A) à stimuler et à perfuser. Les tissus T biologiques obtenus après culture sont destinés à être stimulés mécaniquement grâce au dispositif.

Cette membrane 2_1, 2_2 de culture est déformable. Dans son état repos, elle présente une forme plane, et dans un état déployé, elle est apte à s'expanser ou se gonfler vers le haut, en direction de l'extérieur du puits.

Sur les figures 1A à 1C, cette membrane 2_1 de culture peut être une bande de matière réalisée en deux dimensions (c'est-à-dire avec une épaisseur très faible au regard de ses autres dimensions) et perforée pour pouvoir être traversé par un milieu de culture M nécessaire à la perfusion des tissus T supportés par sa face supérieure. Dans cette configuration, la membrane peut être réalisée dans un matériau tel que le PDMS (PolyDiMéthylSiloxane).

Préférentiellement, comme illustré par les figures 2A à 2C, la membrane 2_2 de culture utilisée est une mousse poreuse tridimensionnelle. Elle comporte ainsi une ou plusieurs rangées de cellules ouvertes superposées. Sa porosité permet aux cellules des tissus T cultivés de venir s'ancrer dans les pores de la mousse, évitant ainsi un décollement des tissus T lors de la stimulation mécanique. Dans cette réalisation, la mousse formant la membrane 2_2 de culture présente une épaisseur constante selon la direction principale. Elle est découpée dans les deux autres directions pour venir s'intégrer dans le composant et obturer la cavité vers le haut.

De manière non limitative, cette mousse peut être réalisée dans un matériau choisi parmi le polyéthylène (PE), le polyuréthane (PU), le Polychlorure de Vinyle (PVC), le caoutchouc ou matériaux équivalents. A titre d'exemple, la mousse en polyéthylène permet une déformation élastique importante (plus de 100% d'élasticité), tout en disposant d'une porosité suffisante pour laisser passer le milieu de culture.

La mousse présente également l'avantage de pouvoir être fabriquée très facilement, avec la porosité voulue et les dimensions adaptées au dispositif de stimulation mécanique.

L'utilisation d'une mousse permet d'obtenir les avantages suivants :
- Elle peut servir de réservoir de milieu ou de divers réactifs ;
- Sa porosité peut être facilement choisie et pas nécessairement uniforme sur tout son volume ;
- Elle peut être facilement fabriquée à bas-coût dans des matériaux biocompatibles ;
- Elle sert de zone d'accroche pour les tissus biologiques à stimuler. L'utilisation d'une membrane poreuse tridimensionnelle (une mousse) permet en effet une prolifération cellulaire en partie à l'intérieur de la mousse ce qui permet un meilleur accrochage du tissu cellulaire lors de la déformation mécanique.

Le composant 1 comporte également un premier circuit fluidique 11 ayant une première cavité 110 réalisée dans le puits 10, sous la membrane 2_1, 2_2 de culture. Ce premier circuit fluidique 11 est dédié à la circulation du milieu de culture 3, injecté sous forme liquide à l'intérieur de la première cavité 110. La face inférieure de la membrane 2_1, 2_2 de culture vient ainsi au contact du milieu de culture 3 présent dans la cavité 110. Le milieu de culture 3 peut se diffuser à travers la membrane 2_1, 2_2 de culture pour venir perfuser les tissus T déposés sur la face supérieure de la membrane 2_1, 2_2 de culture.

Le dispositif comporte également une deuxième membrane 4 déformable et non poreuse, dite membrane 4 d'actionnement. Cette membrane 4 d'actionnement est intégrée au composant 1 de manière à venir fermer la première cavité 110 par le bas, de manière étanche, et former ainsi le fond de ladite première cavité 110.

Elle peut être réalisée dans un matériau très élastique, par exemple un matériau à base de silicone (par exemple une membrane Ecoflex - marque déposée). Une telle membrane 4 est capable de se déformer élastiquement (à plus de 100% par rapport à sa configuration initiale) sans risque de rupture et sans compromettre l'étanchéité du dispositif.

Selon un aspect particulier de l'invention, la membrane 4 d'actionnement est actionnée à l'aide d'un deuxième circuit fluidique 12, avantageusement intégré au composant 1. Ce deuxième circuit fluidique 12 comporte par exemple une deuxième cavité 120 réalisée dans le composant 1, sous la membrane d'actionnement 4. Un fluide 5 peut ainsi être injecté dans ladite deuxième cavité 120, une augmentation de la pression du fluide 5 dans la deuxième cavité 120 entraînant une déformation vers le haut de la membrane 4 d'actionnement.

La membrane 2_1, 2_2 de culture et la membrane 4 d'actionnement sont situées dans un même axe, parallèle à la direction principale X, et sont toutes deux agencées transversalement pour recouvrir la section du puits 10 à deux hauteurs différentes. Elles sont situées l'une au-dessus de l'autre, la membrane 4 d'actionnement étant positionnée sous la membrane 2_1, 2_2 de culture, séparée de celle-ci par la première cavité 110 (figure 1B et figure 2B). La membrane 2_1, 2_2 de culture ferme la première cavité 110 par le haut et la membrane 4 d'actionnement ferme la première cavité 110 par le bas. La première cavité 110 est située entre les deux membranes.

Selon un autre aspect de l'invention, la déformation de la membrane 2_1, 2 2 de culture est obtenue par l'exercice d'une contrainte mécanique directe de la membrane 4 d'actionnement. Autrement dit, la membrane 4 d'actionnement est déformée suffisamment pour venir au contact physique de la membrane 2_1, 2_2 de culture et la pousser de manière à la déformer. La déformation de la membrane 2_1, 2_2 de culture est ensuite transmise aux tissus T biologiques qui sont placés sur sa face supérieure (figure 1C et figure 2C). La déformation de la membrane 4 d'actionnement est permise par la présence de la première cavité 110 dans laquelle est placé le milieu de culture 3. Comme la membrane 4 d'actionnement vient pousser la membrane 2_1, 2_2 de culture en se déployant dans la première cavité 110, le volume occupé par le milieu de culture 3 dans la première cavité 110 est moins important et peut être quasi-nul si la membrane 4 d'actionnement déformée vient épouser la forme de la face inférieure de la membrane 2_1, 2_2 de culture. Pour permettre l'évacuation du milieu de culture 3 lors de l'actionnement, et permettre à la membrane 4 d'actionnement de pousser la membrane 2_1, 2_2 de culture et de venir en contact physique avec celle-ci, il est nécessaire de prévoir au moins un canal d'évacuation du milieu de culture dans le premier circuit fluidique 11, vers l'extérieur de la première cavité 110.

De manière avantageuse, le fluide 5 injecté dans ce deuxième circuit fluidique 12 est un liquide non compressible. L'utilisation d'un liquide permet de mieux régler la déformation de la membrane 4 d'actionnement et donc la stimulation mécanique appliquée aux tissus T, via la membrane 2_1, 2_2 de culture. Bien entendu, l'utilisation d'un gaz pour réaliser un actionnement pneumatique serait également envisageable.

Il faut noter que la déformation mécanique appliquée lors de la culture est une déformation cyclique de l'ordre de quelques déformations par minute à quelques déformations par seconde. A titre d'exemple, l'amplitude de la déformation est de l'ordre de 5% à 50%, réalisée de manière précise et reproductible d'un cycle à l'autre.

Le dispositif de l'invention utilise ainsi un mécanisme à deux membranes : une membrane poreuse et une membrane non poreuse. Ce principe à double-membrane présente certains avantages, parmi lesquels :
- Une déformation mécanique maîtrisée et reproductible, notamment grâce à l'utilisation d'un actionnement à l'aide d'un liquide non-compressible ;
- Une solution d'actionnement indépendante de la culture des tissus T biologiques ;
- Une solution qui limite les risques de décollement des tissus T biologiques lors de la stimulation ;

Une variante de réalisation est décrite ci-dessous en liaison avec les figures 3A à 3C. Dans cette variante, les références utilisées pour les réalisations précédentes sont conservées dans la mesure où les éléments sont identiques.

La figure 3A illustre le dépôt de la culture cellulaire ECH dans le puits 10 du composant fluidique 1.

Selon cette variante, la mousse tridimensionnelle utilisée pour réaliser la membrane 2_3 de culture est structurée. Par structuration, on entend que la mousse peut comporter des variations d'épaisseur, suivant la direction principale (X) (figure 3B). Ces variations d'épaisseur se matérialisent par exemple par des creux 230 ou des pointes ou plots 231 sur sa face supérieure. Lorsque la membrane 2_3 de culture est déformée (figure 3C), par contrainte mécanique directe de la membrane 4 d'actionnement, la structuration de sa face supérieure permet d'appliquer différents niveaux d'étirement aux tissus T biologiques stimulés.

Les autres caractéristiques et avantages des solutions décrites ci-dessus restent valables pour cette dernière réalisation.

Il faut noter que des essais concluants ont été menés pour s'assurer que des tissus T biologiques déposés sur une mousse peuvent être bien perfusés à travers la mousse par un milieu de culture.

Il faut noter que chaque circuit fluidique 11, 12 comporte des vannes, permettant de contrôler l'apport de fluide dans la première cavité 110 et la deuxième cavité 120. Ce mécanisme de vanne, associé à la membrane 4 d'actionnement, peut notamment être utilisé pour faire fonctionner le dispositif comme une pompe, permettant le renouvellement du milieu de culture 3 :
- On place ainsi une première vanne en amont de la première cavité 110 et une deuxième vanne en aval de la première cavité 110.
- Lors de la stimulation mécanique, on ferme la première vanne et on ouvre la deuxième vanne, la déformation de la membrane 4 d'actionnement pousse alors le milieu de culture 3 vers l'aval, ce qui permet de pousser mécaniquement la membrane 2_1, 2_2 de culture.
- La deuxième vanne est fermée. La membrane 4 d'actionnement est alors toujours dans son état déployé.
- Puis on ouvre la première vanne. Et on retire la contrainte sur la membrane 4 d'actionnement. En retournant vers l'état repos, la membrane 4 d'actionnement pompe le liquide du milieu de culture 3 vers l'intérieur de la première cavité 110. On renouvelle ainsi facilement le milieu de culture.

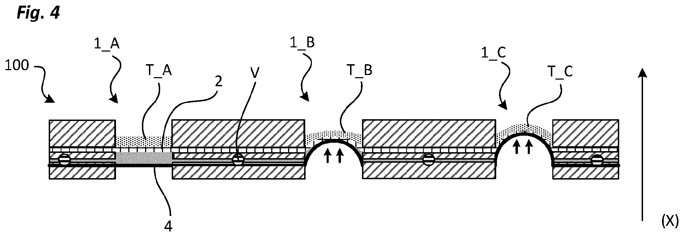

De manière avantageuse, comme illustré par la figure 4 le dispositif de l'invention peut être dupliqué, par exemple au sein d'un même composant, avec plusieurs dispositifs 1_A, 1_B, 1_C juxtaposés, ce qui permet d'obtenir un système 100 complet. On peut ainsi utiliser une seule membrane 4 d'actionnement et une seule membrane 2 de culture, commune à tous les dispositifs. La membrane de culture est par exemple la mousse utilisée dans la variante des figures 2A à 2C. Elle pourrait aussi être structurée, au moins partiellement, par exemple au niveau d'un seul dispositif parmi l'ensemble des dispositifs juxtaposés. Des vannes V sont par exemple placées sur le premier circuit fluidique 11 pour contrôler les apports du milieu de culture 3. Un actionnement indépendant de la membrane 4 d'actionnement au niveau de chaque dispositif peut être prévu pour pouvoir éventuellement assurer des niveaux de stimulation distincts d'un dispositif à un autre.

Et grâce à ce système, on peut ainsi (les options ci-dessous pouvant être combinées entre elles) :
- Placer des échantillons différents d'un dispositif à un autre ;
- Utiliser différentes mousses d'un dispositif à un autre, celles-ci pouvant avoir différentes porosités et des structurations différentes ;
- Appliquer des stimulations différentes d'un dispositif à un autre ;
- Appliquer des conditions de culture différentes d'un dispositif à un autre ;
- Appliquer des traitements chimiques différents d'un dispositif à un autre ;

Sur la figure 4, les tissus T_A, T_B, T_C biologiques peuvent être stimulées mécaniquement de manière distincte, dans chaque dispositif 1_A, 1_B, 1_C du système 100.

L'invention décrite ci-dessus sera notamment très pertinente pour la stimulation mécanique de la peau. Il sera également possible de l'utiliser pour un modèle pulmonaire ou intestinal.

## Revendications

1. Dispositif de culture cellulaire doté d'une fonction de stimulation mécanique de tissus (T) biologiques, comprenant :
- Un composant (1) fluidique dans lequel est réalisé un puits (10) qui est creusé selon une direction dite principale (X),
- Une première membrane poreuse déformable élastiquement, dite membrane (2_1, 2_2, 2_3) de culture, intégrée audit composant et s'étendant transversalement à ladite direction principale (X) pour obturer ledit puits, ladite membrane de culture comprenant une face supérieure destinée à recevoir une culture cellulaire et une face inférieure opposée,
- Un premier circuit fluidique (11) intégré audit composant, comprenant une première cavité (110) réalisée dans ledit puits sous ladite membrane (2_1, 2_2, 2_3) de culture et destinée à recevoir un milieu de culture (3) adapté à la perfusion desdits tissus (T) biologiques,
- Une deuxième membrane non poreuse, dite membrane (4) d'actionnement, déformable élastiquement, intégrée audit composant (1),
- Des moyens d'actionnement de ladite membrane (4) d'actionnement,
- **Caractérisé en ce que** :
- La membrane (4) d'actionnement est agencée de manière à fermer le fond de ladite première cavité (110) de manière étanche, et configurée pour se déformer sous l'action desdits moyens d'actionnement vers une position dans laquelle elle pousse mécaniquement ladite membrane (2_1, 2_2, 2_3) de culture pour la déformer.

2. Dispositif selon la revendication 1, **caractérisé en ce que** la membrane (2_2, 2_3) de culture est une mousse tridimensionnelle d'épaisseur constante suivant la direction principale (X).

3. Dispositif selon la revendication 2, **caractérisé en ce que** la mousse tridimensionnelle est structurée sur sa face supérieure de manière à présenter des variations d'épaisseur suivant la direction principale (X).

4. Dispositif selon la revendication 2 ou 3, **caractérisé en ce que** la mousse est réalisée dans un matériau choisi parmi le polyéthylène, le polyuréthane, le polychlorure de vinyle, le caoutchouc.

5. Dispositif selon la revendication 1, **caractérisé en ce que** la membrane (2_1) de culture est une bande de matière perforée, agencée transversalement à ladite direction principale.

6. Dispositif selon la revendication 5, **caractérisé en ce que** la membrane (2_1) est réalisée en PDMS.

7. Dispositif selon l'une des revendications 1 à 6, **caractérisé en ce que** les moyens d'actionnement de la membrane (4) d'actionnement comportent un deuxième circuit fluidique (12) d'actionnement, intégré audit composant et comprenant au moins une deuxième cavité (120) de mise en pression située sous la membrane (4) d'actionnement et destinée à recevoir un fluide (5).

8. Système de culture cellulaire, **caractérisé en ce qu'**il comporte plusieurs dispositifs de culture cellulaire à fonction de stimulation mécanique tels que définis dans l'une des revendications 1 à 7, lesdits dispositifs étant juxtaposés au sein d'un même composant fluidique.

## Patentansprüche

1. Zellkulturvorrichtung, die über eine Funktion der mechanischen Stimulation biologischer Gewebe (T) verfügt, umfassend:
- ein Fluidmodul (1), das mit einer Vertiefung (10) versehen ist, die sich entlang einer sogenannten Hauptrichtung (X) erstreckt;
- eine erste elastisch verformbare poröse Membran, die als Kulturmembran (2_1, 2_2, 2_3) bezeichnet wird, wobei sie fester Bestandteil des Moduls ist und sich quer zur Hauptrichtung (X) erstreckt, um die Vertiefung zu verschließen, wobei die Kulturmembran eine Oberseite, die für die Aufnahme einer Zellkultur bestimmt ist, und eine gegenüberliegende Unterseite umfasst;
- einen ersten Fluidkreislauf (11), der fester Bestandteil des Moduls ist und einen ersten Hohlraum (110) umfasst, der sich in der Vertiefung unter der Kulturmembran (2_1, 2_2, 2_3) befindet und dazu bestimmt ist, ein Kulturmedium (3) aufzunehmen, welches sich zur Perfusion des biologischen Gewebes (T) eignet;
- eine zweite nicht poröse Membran, die als elastisch verformbare Betätigungsmembran (4) bezeichnet wird und fester Bestandteil des Moduls (1) ist,
- Mittel zum Betätigen der Betätigungsmembran (4),
- **dadurch gekennzeichnet, dass**
- die Betätigungsmembran (4) derart angeordnet ist, dass sie den Boden des ersten Hohlraums (110) abdichtend verschließt, wobei sie dafür ausgelegt ist, sich unter Einwirkung der Betätigungsmittel in Richtung einer Position zu verformen, in welcher sie mechanischen Druck auf die Kulturmembran (2_1, 2_2, 2_3) ausübt, um diese zu verformen.

2. Vorrichtung nach Anspruch 1, **dadurch gekennzeichnet, dass** es sich bei der Kulturmembran (2_2, 2_3) um einen dreidimensionalen Schaumstoff handelt, dessen Dicke gemäß der Hauptrichtung (X) konstant ist.

3. Vorrichtung nach Anspruch 2, **dadurch gekennzeichnet, dass** der dreidimensionale Schaumstoff auf seiner Oberseite derart strukturiert ist, dass seine Dicke gemäß der Hauptrichtung (X) variiert.

4. Vorrichtung nach Anspruch 2 oder 3, **dadurch gekennzeichnet, dass** der Schaumstoff aus einem Material hergestellt ist, das aus Polyethylen, Polyurethan, Polyvinylchlorid und Gummi ausgewählt ist.

5. Vorrichtung nach Anspruch 1, **dadurch gekennzeichnet, dass** er sich bei der Kulturmembran (2_1) um einen perforierten Materialstreifen handelt, der quer zur Hauptrichtung angeordnet ist.

6. Vorrichtung nach Anspruch 5, **dadurch gekennzeichnet, dass** die Membran (2_1) aus PDMS hergestellt ist.

7. Vorrichtung nach einem der Ansprüche 1 bis 6, **dadurch gekennzeichnet, dass** die Mittel zur Betätigung der Betätigungsmembran (4) einen zweiten Betätigungsfluidkreislauf (12) aufweisen, der fester Bestandteil des Moduls ist und mindestens einen zweiten Hohlraum (120) zum Druckaufbau umfasst, welcher sich unter der Betätigungsmembran (4) befindet und dazu bestimmt ist, ein Fluid (5) aufzunehmen.

8. Zellkultursystem, **dadurch gekennzeichnet, dass** es mehrere Zellkulturvorrichtungen mit einer mechanischen Stimulationsfunktion gemäß der Begriffsbestimmung in einem der Ansprüche 1 bis 7 umfasst, wobei die Vorrichtungen innerhalb ein und desselben Fluidmoduls nebeneinander angeordnet sind.

## Claims

1. Cell culture device with a function for the mechanical stimulation of biological tissues (T), comprising:
- A fluidic component (1) in which a well (10) is made which is dug in a "main" direction (X),
- A first elastically deformable porous membrane, referred to as a culture membrane, (2_1, 2_2, 2_3), integrated into said component and extending transversely to said main direction (X) to close off said well, said culture membrane comprising an upper face intended to receive a cell culture and an opposite lower face,
- A first fluidic circuit (11) integrated into said component, comprising a first cavity (110) made in said well under said culture membrane (2_1, 2_2, 2_3) and intended to receive a culture medium (3) suitable for infusing said biological tissues (T),
- A second elastically deformable non-porous membrane, referred to as the actuating membrane (4), integrated into said component (1),
- Means for actuating said actuating membrane (4),
- **Characterized in that**:
- The actuating membrane (4) is arranged to tightly seal the bottom of said first cavity (110), and configured to deform under the action of said actuating means to a position in which it mechanically pushes said culture membrane (2_1, 2_2, 2_3) to deform it.

2. Device according to Claim 1, **characterized in that** the culture membrane (2_2, 2_3) is a three-dimensional foam of constant thickness in the main direction (X).

3. Device according to Claim 2, **characterized in that** the three-dimensional foam is structured on its upper face so as to have variations in thickness in the main direction (X).

4. Device according to Claim 2 or 3, **characterized in that** the foam is made of a material chosen from polyethylene, polyurethane, polyvinyl chloride and rubber.

5. Device according to Claim 1, **characterized in that** the culture membrane (2_1) is a perforated strip of material arranged transversely to said main direction.

6. Device according to Claim 5, **characterized in that** the membrane (2_1) is made of PDMS.

7. Device according to one of Claims 1 to 6, **characterized in that** the means for actuating the actuating membrane (4) include a second actuating fluidic circuit (12), integrated into said component and comprising at least one second pressurization cavity (120) located under the actuating membrane (4) and intended to receive a fluid (5).

8. Cell culture system, **characterized in that** it includes several cell culture devices with a mechanical stimulation function as defined in one of Claims 1 to 7, said devices being juxtaposed within the same fluidic component.
